# EUROPEAN PATENT APPLICATION

(11) **EP 4 046 689 A1**
(43) Date of publication of application: **24.08.2022**
(21) Application number: 21157812.5
(22) Date of filing: 18.02.2021
(51) Int. Cl.: A61P 31/14, A61K 35/76, C12N 7/04

(54) **INFLUENZA VIRUS DEFECTIVE INTERFERING PARTICLES FOR USE IN THE PROPHYLACTIC OR THERAPEUTIC TREATMENT OF CORONAVIRIDAE INFECTION**

(71) Applicant: Max-Planck-Gesellschaft zur Förderung der Wissenschaften e.V., 80539 München (DE); Helmholtz-Zentrum für Infektionsforschung GmbH, 38124 Braunschweig (DE); Otto-von-Guericke-Universität Magdeburg, 39106 Magdeburg (DE)
(72) Inventor: KUPKE, Sascha, 39167 Hohe Börde (DE); REICHL, Udo, 39104 Magdeburg (DE); HEIN, Marc Dominique, 13156 Berlin (DE); BRUDER, Dunja, 38173 Lucklum (DE); RAND, Ulfert, 38124 Braunschweig (DE)
(74) Representative: Gramm, Lins & Partner Patent- und Rechtsanwälte PartGmbB

(57) **Abstract**

The present invention relates in a first aspect to the use of influenza virus defective interfering particles in the prophylactic or therapeutic treatment of *coronaviridae* infection. In particular, it has been recognized that defective interfering particles of influenza A have beneficial effects in the treatment of SARS coronavirus infection, in particular, SARS-CoV-2 infection. Further, the present invention relates to a pharmaceutical composition for use in treating *coronaviridae* infection, in particular SARS-CoV-2.

SEQUENCE LISTING

<110> Max-Plank-Gesellschaft zur Foerderung der Wissenschaften
e.V.
Otto-von-Guericke-Universitaet Magdeburg
Helmholtz-Zentrum fuer Infektionsforschung GmbH

<120> Influenza virus defective interfering particles for use in the
prophylactic or therapeutic treatment of coronaviridae infection

<130> 4188-0022 EP-1

<160> 4

<170> PatentIn version 3.5

<210> 1
<211> 395
<212> DNA
<213> influenza A virus

<400> 1
agtagaaaca aggtcgtttt taaactattc gacactaatt gatggccatc cgaattcttt 60

tggtcgctgt ctggctgtca gtaagtatgc tagagtcccg tttccgtttc attaccaaca 120

ccacatcccc ttgcccaatt agcacattag ccttctctcc tttcgcaagg ttgctcagtt 180

cattgatgct tagtgctggc ccatatctct tgtcctcttt gcccagaatg aggaatcccc 240

tcagtcttct cctgtcttcc tgatgtgtac ttcttgatta tggccatatg gtccacggtg 300

gtttttgtga gtatctcgcg ggtgcgagac tgcgacatta gatttcttag ttcttttatt 360

ctttccatat tgaatatatt tgacctgctt tcgct 395

<210> 2
<211> 395
<212> DNA
<213> Influenza A virus

<400> 2
agtagaaaca aggtcgtttt taaactattc gacactaatt gatggccatc cgaattcttt 60

tggtcgctgt ctggctgtca gtaagtatgc tagagtcccg tttccgtttc attaccaaca 120

ccacatcccc ttgcccaatt agcacattag ccttctctcc tttcgcaagg ttgctcagtt 180

cattgatgct tagtgctggc ccatatctct tgtcctcttt gcccagaatg aggaatcccc 240

tcagtcttct cctgtcttcc tgatgtgtac ttcttgatta tggccatatg gtccacggtg 300

gtttttgtga gtatctcgcg ggtgcgagac tgcgacatta gatttcttag ttcttttatt 360

ctttccatat tgaatataat tgacctgctt tcgct 395

<210> 3
<211> 1027
<212> DNA
<213> Influenza Avirus

<400>  3
agtaaaaaca ggtagatgtt gaaagatgag tcttctaacc gaggtcgaaa cgtacgttct      60

ctctatcatc ccgtcaggcc ccctcaaagc cgagatcgcg cagagacttg aaaatgtctt     120

tgcagggaag aacaccgatc ttgaggttct catggaatgg ctaaagacaa gaccaatcct     180

gtcacctctg actaagggga ttttaggatt tgtgttcacg ctcaccgtgc ccagtgagca     240

gggactgcag cgtagacgct ttgtccaaaa tgcccttaat gggaacgggg atccaaataa     300

catggacaaa gcagttaaac tgtataggaa gcttaagagg gagataacat tctatggggc     360

taaagaaata gcactcagtt attccgctgg tgcacttgcc tgttgtatgg gcctcatata     420

caacaggatg gggactgtga ctgctgaagt ggtgtttggc ctggtatgtg caacctgtga     480

acagattgct gactcccagc atcggtctca taggcaaatg gtggcaacaa ccaatccact     540

aatcagacat gagaacagaa tggttttagc cagcactaca gctaaggcta tggagcaaat     600

ggctggatcg agtgagcaag cagcagaggc catggaggtt gcgattcggg ctaggcaaat     660

ggtgcaggca atgagaacca ttgggactca tcctagctcc agtgctggtc tgaaagatga     720

tcttcttgaa aatttgcagg cctatcagaa acgaatgggg gtgcagatgc aacggttcaa     780

gtgatcctct cgttattgcc tcaagtatca ttgggatctt gcacttgata ttgtggattc     840

ttgatcgtct ttttttcaaa tgcatttacc gtctctttaa atacggtttg aaaagagggc     900

cttctacgga aggagtgcca aagtctatga gggaagaata tcaaaaggaa cagcagagtg     960

ctgtggatgc tgacgatggt cattttgtca gcatagagct ggagtaaaaa actaccttgt    1020

ttctact                                                              1027


<210>  4
<211>  1027
<212>  DNA
<213>  Influenza A virus

<400>  4
agtaaaaaca ggtagatatt gaaagatgag tcttctaacc gaggtcgaaa cgtacgttct      60

ctctatcatc ccgtcaggcc ccctcaaagc cgagatcgcg cagagacttg aaaatgtctt     120

tgcagggaag aacaccgatc ttgaggttct catggaatgg ctaaagacaa gaccaatcct     180

gtcacctctg actaagggga ttttaggatt tgtgttcacg ctcaccgtgc ccagtgagca     240

gggactgcag cgtagacgct ttgtccaaaa tgcccttaat gggaacgggg atccaaataa     300

catggacaaa gcagttaaac tgtataggaa gcttaagagg gagataacat tctatggggc     360

taaagaaata gcactcagtt attccgctgg tgcacttgcc tgttgtatgg gcctcatata     420

caacaggatg gggactgtga ctgctgaagt ggtgtttggc ctggtatgtg caacctgtga     480

acagattgct gactcccagc atcggtctca taggcaaatg gtggcaacaa ccaacccact     540

aatcagacat gagaacagaa tggttttagc cagcactaca gctaaggcta tggagcaaat     600

ggctggatcg agtgagcaag cagcagaggc catggaggtt gcgattcggg ctaggcaaat 660

ggtgcaggca atgagaacca ttgggactca tcctagctcc agtgctggtc tgaaagatga 720

tcttcttgaa aatttgcagg cctatcagaa acgaatgggg gtgcagatgc aacggttcaa 780

gtgatcctct cgttattgcc tcaagtatca ttgggatctt gcacttgata ttgtggattc 840

ttgatcgtct ttttttcaaa tgcatttacc gtctctttaa atacggtttg aaaagagggc 900

cttctacgga aggagtgcca aagtctatga gggaagaata tcaaaaggaa cagcagagtg 960

ctgtggatgc tgacgatggt cattttgtca gcatagagct ggagtaaaaa actaccttgt 1020

ttctact 1027

## Description

The present invention relates in a first aspect to the use of influenza virus defective interfering particles in the prophylactic or therapeutic treatment of *coronaviridae* infection. In particular, it has been recognized that defective interfering particles of influenza A virus have beneficial effects in the treatment of SARS coronavirus infection, in particular, SARS-CoV-2 infection. Further, the present invention relates to a pharmaceutical composition for use in prophylaxis and treatment of *coronaviridae* infection, in particular SARS-CoV-2 as well as compositions or a kit comprising defective interfering particles of influenza A virus to form a therapeutic pharmaceutical or for use in treating or protecting against said virus infection by pre- and/or post exposure treatment.

### Prior art

Defective interfering particles (DIPs) of influenza A virus (IAV) usually contain a large internal deletion in one of the eight genomic viral RNAs (vRNAs). This results in a defect in virus replication, which can be complemented by co-infection with fully infectious standard virus (STV). In addition, DIPs carrying mutations have been described, e.g. DIP OP7, see KUPKE, S. Y., et. al., 2019. J Virol, ;93(4):e01786-18. doi: 10.1128/JVI.01786-18. Furthermore, DIPs specifically interfere with homologous STV replication and spreading in a co-infection scenario which is also known as replication interference.

IAV DIPs were previously proposed for antiviral treatment against the flu (e.g. ZHAO, H., et. al., 2018. Nat Commun, 9, 2358), but also for pan-specific treatment of other respiratory viral diseases (e.g. DIMMOCK, N. J. & EASTON, A. J. 2014. J Virol, 88,5217-27). IAV DIPs carry typically a large internal deletion or alternatively recently discovered point mutations (Kupke et al., 2019, see above), in their genome, rendering them defective in virus replication (e.g. ALNAJI, F. G. & BROOKE, C. B. 2020 PLoS Pathog, 16, e1008436). Furthermore, they suppress and interfere specifically with homologous viral replication and spreading in co-infection scenario. As a result, administration of IAV DIPs to mice resulted in full protection against a lethal dose of IAV (e.g. DIMMOCK, N. J., 2012 PLoS One, 7, e49394, HEIN, M. D., et. al., 2021, Appl Microbiol Biotechnol, Jan; 105(1):129-146). In the ferret model, IAV DIP co-treatment resulted in a reduced severity of clinical flu disease (e.g. DIMMOCK, N. J., et. al., 2012, PLoS One, 7, e49394). Intriguingly, mice were also protected against a lethal infection with the unrelated influenza B virus and pneumonia virus of mice, a pneumovirus from the family *Paramyxoviridae*, mediated by other mechanisms, e.g. the ability of IAV DIPs to stimulate innate immunity.

The recently identified severe acute respiratory syndrome coronavirus 2 (SARS-CoV-2), causing coronavirus disease 2019 (COVID-19), poses a severe burden to public health, economy and society. To date, more than two million cases of deaths were reported in the context of SARS-CoV-2 infection (WHO, covid19.who.int). In 2020, there has been an unprecedented race for the development of (novel) vaccines, their production, and safety and immunogenicity studies in clinical trials. First individuals were already treated with an approved vaccine at the end of 2020. While vaccination typically provides the best protection against virus infections, the production capacity of COVID-19 vaccines, and the infrastructure required for vaccination is still limiting. In addition, mutations arised rendering the efficacy of various vaccine candidates questionable. An alternative treatment option is the use of antivirals. Yet, remdesivir (in clinical use) shows only limited efficacy (e.g. WANG, Y., et. al., 2020. Lancet, 395, 1569-1578Wang et al., 2020), while other repurposed drug candidates (e.g. hydroxychloroquine and lopinavir-ritonavir) showed a lack of efficacy. In addition, cocktails of monoclonal antibodies (e.g. bamlanivimab (CHEN, P.,et. a., 2020, N Engl J Med)) as well as corticosteroids (i.e. dexamethasone (Tomazini e OMAZINI,et. al., 2020, JAMA, 324, 1307-1316)) are being used in the clinic and show an antiviral effect. Yet, the commonality of the available antiviral intervention options is the amelioration, but not the prevention of disease; therefore, there is an urgent need of new treatment modalities for infections with coronaviridae, including COVID, like COVID-19.

### Brief description of the present invention

The present inventions relates to influenza virus defective interfering particles for prophylactic and treatment of *coronaviridae* infection, in particular, SARS-CoV-2 infection. The present inventors surprisingly recognized that treatment with these influenza virus defective interfering particles (IVDIP) have beneficial effects in terms of abrogating SARS-CoV-2 replication in a human cell culture model, thus, an effective anti-viral treatment of corona infection, in particular, COVID-19 is possible.

It was shown that SARS-CoV-2 infection results in modulation and inhibition of the interferon (IFN) response; yet, on the other hand, replication was susceptible to inhibition by exogenously added IFNs *in vitro* and in clinical trials (e.g. CHEN, D. Y., et. al., 2020. bioRxiv, doi: https://doi.org/10.1101/2020.10.27.358259, MONK, et. al., 2020, Lancet Respir Med, 9: 196-206.Monk et al., 2020). Therefore, the inventors investigated whether IAV DIPs would also be able to suppress SARS-CoV-2 replication through their ability to stimulate a type I IFN response and thus an antiviral state in target cells. Previously, a cell culture-based, manufacturing workflow in a laboratory-scale stirred tank bioreactor for production of IAV DIPs was developed (Hein et al., 2020, see above). Specifically, two promising candidate DIPs: a prototypic, well-characterized conventional IAV DIP "DI244" (DIMMOCK, N. J. & EASTON, A. J. 2014. J Virol, 88, 5217-27), and the newly discovered "OP7" (Kupke et al., 2019, see above) were produced. The manufactured DIPs showed no apparent toxic effects upon administration to mice, and a potent antiviral activity. Further, their administration resulted in a rescue of mice from a lethal challenge with IAV (Hein et al., 2020, see above). In addition, for DI244 (but not OP7), we used a novel system based on a genetically modified cell line for production of purely clonal DIPs (BDEIR, N., et. al., 2019, PLoS One, 14, e0212757Bdeir et al., 2019), abrogating the need for UV-irradiation to inactivate infectious helper virus.

Herein Calu-3 cells (human lung cancer cell line) were used for *in vitro* co-infection experiments with SARS-CoV-2 and DI244, or OP7, respectively. Both DIPs were able to completely shut down SARS-CoV-2 replication, similar as compared to IFN-β or remdesivir treatment. Moreover, it is shown that the inhibitory effect of IAV DIPs was caused by their ability to induce innate immunity. Most likely, other mechanisms interfering with IAV replication and spreading also play a role. Thus, IAV DIPs are effective antiviral agents for COVID-19, like for the treatment of early stage COVID-19. Furthermore, the use of IAV DIPs as potential universal antiviral agents, not only against different IAV subtypes, but also against other IFN-sensitive respiratory viruses was demonstrated.

Namely, in a first aspect the present invention relates to an influenza virus defective interfering particle (DIP) for use in the prophylactic or therapeutic treatment of *coronaviridae* infection.

As mentioned, beneficial effects of influenza virus DIP (IVDIP) have been shown against a different family of virus, namely, the *coronaviridae* virus family.

In particular, the present invention relates to the use of the influenza A virus DIPs in the prophylactic or therapeutic treatment of *coronaviridae* infection.

### Brief description of the drawings

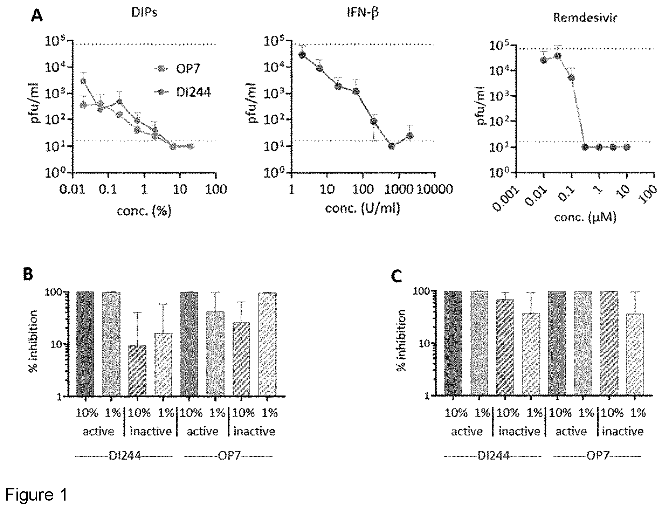

Figure 1: **Inhibition of SARS-CoV-2 replication by IAV DIPs.** SARS-CoV-2-infected Calu-3 cells were treated with IAV DIPs (DI244 and OP7), IFN-β, or remdesivir at 1 hpi (hours post infection). For DI244 and OP7 treatment, highly concentrated cell culture-manufactured DIP material was used, where % (v/v) indicates the fraction with respect to the total cell culture volume used. Independent experiments were conducted (n=3). (A) Effective concentration range of DI244 and OP7 compared to IFN-β and remdesivir. Viral titres were determined from the supernatant at 3 day post infection (dpi) by plaque assay. Mean +/-SD is depicted. (B) and (C) SARS-CoV-2 growth inhibition by inactivated DIPs. SARS-CoV-2 infected cells were treated with active or UV-inactivated DIPs at 1 hpi (B) or 24 hpi (C). Percent inhibition of viral growth relative to mock treatment (mean +/- SEM) is depicted.

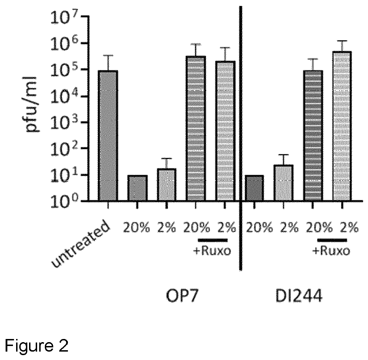

Figure 2: **Suppression of SARS-CoV-2 replication by IAV DIPs under JAK inhibition.**
   SARS-CoV-2-infected Calu-3 cells were treated with IAV DIPs (DI244 and OP7) at 1 hpi in the presence of absence of ruxolitinib (JAK inhibitor). % (v/v) indicates the fraction of DIPs (highly concentrated material from cell culture-based manufacturing (Hein et al., 2020) with respect to the total cell culture volume used. Viral titres were determined from the supernatant at 3 dpi by plaque assay. Independent experiments were conducted (n=3), mean +/-SD is depicted.

### Detailed description of the invention

In a first aspect, the present invention relates to an influenza virus defective interfering particle (DIP) for use in the prophylactic or therapeutic treatment of *coronaviridae* infection in an individual.

Influenza virus DIPs (IVDIPs) demonstrate beneficial effects in reducing coronaviridae infection, like SARS-CoV-2 infection in individuals as demonstrated in cell culture experiments with human lung epithelial cells. That is, surprisingly, DIPs of a different virus family can be used to treat prophylactically and therapeutically viral infections of unrelated species and families, here the coronaviridae family including the genus of sarbecovirus, like SARS-CoV and SARS-CoV-2 as causative agent of COVID-19 disease.

As used herein, the term "particle(s)" includes virus-like particle(s), viral vector(s) and virus particle(s) unless otherwise indicated. The particles contain nucleic acid molecules encoding at least part of the respective virus.

The term "viral vector" refers to recombinant viral particles.

The term "virus particle" refers to whole virus being infectious unless attenuated or inactivated.

As used herein, the term "virus-like particle" (VLP) refers to particles comprising the lipids and proteins of the envelope or proteins of the capsid and, in addition, may contain additional genetic material. That is, it may contain at least one nucleic acid molecule, namely, at least the nucleic acid molecules described herein. VLP are non-infectious particles. VLP include DIPs as described, also referred to as DI virus.

Of course, the virus-like particles may contain other nucleic acid molecules. For example, in case of influenza virus, the virus-like particle or viral vector contains the different segments of genetic material in form of RNA, e.g. in form of the viral ribonucleoprotein (vRNP) complex that comprises the viral RNA and the viral proteins like PB2, PB1, PA and NP (allowing protection against RNA degradation), while the viral capsid or viral envelope is composed of proteins derived from influenza A or other virus including influenza B and influenza C.

The virus defective interfering particles, are characterized in being non-infectious and typically replicate only when its genome is present in a cell which has been infected by a virus with a complete genome. DIPs have the ability to inhibit replication and spreading of infectious virus. Typically, DIPs have mutations resulting in deletions in the viral genome or point mutations resulting in defective virus replication, eventually not producing progeny virions. However, they suppress STV replication and spreading in cell cultures whereby non-infectious DIPs are released but very low or none infectious STV.

According to the present invention, virus DIPs, including the virus-like particle or the viral vector are derived from influenza virus, in particular, influenza A virus. In an embodiment of the present invention, the DIPs, like viral vector or virus particles, are influenza A virus, (IVADIPs) containing nucleic acid sequence containing deletions or point mutations, in particular, the nucleic acid sequence being an RNA-molecule of SEQ ID No. 3 as segment 7 optionally together with the other segments 1 to 6 and 8 of influenza A virus. Alternatively, they contain the nucleic acid sequence of SEQ ID No. 1 or 2 as segment 1 optionally together with the other segments 2 to 8 influenza A virus.

In an embodiment of the present invention, the defective interfering influenza A virus is a virus,
i) wherein the nucleic acid sequence of segment 1 of the influenza A virus comprises:
   a. A sequence selected from SEQ ID No. 1 or SEQ ID No. 2; or
   b. a nucleic acid sequence of at least 99 % identity with SEQ ID No. 1 or SEQ ID No. 2; or
ii) wherein the nucleic acid sequence comprises SEQ ID No. 3 containing the following substituents: C3T, G4A, G8A, A100G, G113A, G130A, G240A, A241G, C334T, C353T, C361T, C370A, T371G, T385C, A401T, G434A, C442T, A443G, C453T, A454G, A524G, T643G, G645T, A648G, A667G, G670A, A716G, C793T, G801T, A805G, G874T, A887T, C888T, G894A, G943A, A18G and C535T, compared to the wild type sequence of SEQ ID No. 4; or a nucleic sequence having greater than 99 % identity with the sequence of SEQ ID No. 3 whereby the above 37 substituents remain present.

In an embodiment of the present invention, the influenza A virus defective interfering particles for use in treating an individual as described herein, are IAV DIPs wherein the nucleic acid sequence comprises SEQ ID No. 3; or a nucleic acid sequence having greater than 99 % identity with the sequence of SEQ ID No. 3.

In an embodiment of the present invention, the nucleic acid sequence is a nucleic acid sequence having SEQ ID No. 3.

In an embodiment of the present invention, the nucleic acid molecule is in form of an RNA, in particular, the isolated nucleic acid molecule represents a protective interfering RNA (piRNA) derived from genome segment 7 of influenza A virus. Sequence ID No. 4 as described above refers to the sequence of NCBI reference sequence: NC_002016.1.

The nucleotide sequence may be nucleotide sequence having greater than 99% identity within the sequence of SEQ ID No. 3 for example, the nucleic acid molecule comprises a nucleotide sequence having greater than 99% identity within the sequence of SEQ ID No. 3 whereby the 37 substituents present in SEQ. ID No. 3 remain present. The nucleotide sequence may have greater than 99%, 99.5%, 99.6%, 99.7%, 99.8% or 99.9% identity with SEQ ID No. 3. Further, the nucleotide sequences may have greater than 99%, 99.5%, 99.6%, 99.7%, 99.8% or 99.9% identity with SEQ ID No. 1, or 2.

In an embodiment, the present invention relates to influenza virus defective interfering particles for use in the prophylactic or therapeutic treatment of *orthocoronaviridae*, in particular, *betacoronaviridae,* infection in an individual.

Further, the present invention relates to influenza virus defective interfering particles for use in preventing or treating infection by *sarbecovirus* virus, in particular, *severe acute respiratory syndrome related coronavirus* or of *merbecovirus* including *Middle East respiratory syndrome related coronavirus.*

Particularly, the present invention describes the use of the DIPs described herein, in particular, the IAVDIPs for use in treating prophylactically or therapeutically individuals against SARS-CoV-2 infection.

In a further aspect, the present invention relates to a pharmaceutical composition containing the DIPs as described herein for use in treating prophylactically or therapeutically individuals against coronaviridae infection as described herein, like infection by *sarbecovirus* virus, in particular, *severe acute respiratory syndrome related coronavirus* or of *merbecovirus* including *Middle East respiratory syndrome related coronavirus,* in particular, SARS-CoV-2 infection.

The pharmaceutical composition is particular useful as a therapeutic, in particular, as an early therapeutic. In particular, the pharmaceutical composition according to the present invention is for use in the treatment of a viral infection pre- or post-exposure to the virus as described herein.
The pharmaceutical composition according to the present invention is adapted for administration to an individual being an animal or human. In an embodiment, the animal is selected from cat, fruit bats, ferrets, dogs or mink. In another embodiment, the individual is a human.

For example, the pharmaceutical composition is adapted for administration by the mucosal route, like the intranasal administration, or orally. The admistation may be to the respiratory tract using known means including nebulizer, droplet spray, etc. Alternatively, the administration may be conducted by intravenous administration, intramuscular administration or subcutaneous administration.

The pharmaceutical composition may contain additionally suitable diluents, carrier or effluence. The skilled person is well aware of suitable diluents, effluents or carriers accordingly.

In an embodiment the pharmaceutical composition according to the present invention is for use in delivering immune response, e.g. stimulation of innate immunity against virus in an individual suffering from infection or being at risk of being infected by *coronaviridae* as described herein, in particular, infection with SARS-CoV2.

In a further embodiment, the present invention relates to a composition or kit comprising an influenza virus defective interfering particles as defined herein, in particular, in form of a pharmaceutical composition, like in form of a therapeutic for use in treating or protecting against a viral infection as defined herein, in particular, SARS-CoV2, e.g. for pre- and post-exposure treatment.

Moreover, an influenza virus defective interfering particles for use according to the present invention is described, wherein said virus particles demonstrate after infection at least one of
i). a reduction in the total number of virus particles,
ii). a severe reduction in the faction of infectious virus produced,
viii). a stronger induction of the innate immune response expressed by type I IFN expression is observable.

Finally, the present invention relates to methods of therapeutically or prophylactically treating viral infection with *coronaviridae* in an individual including administering an effective amount of the DIPs described herein, in particular, the IAVDIPs including DI244 and OP7. In particular, the method according to the present invention relates to the treatment of *orthocoronaviridae,* in particular, *betacoronaviridae.*

For example, the method according to the present invention allows to prevent or treat infection by *sarbecovirus* virus, in particular, *severe acute respiratory syndrome related coronavirus* or of *merbecovirus* including *Middle East respiratory syndrome related coronavirus.* Preferably, treatment is against SARS-CoV-2 infection.

Typically, administration is a mucosal or intramuscular administration of the DIPs including administration to the respiratory tract by known means as described herein.

In addition, the invention further provides a method of therapeutic or prophylactic treatment of an individual against the mentioned *coroviridae* infections including SARS-CoV2 infection, e.g pre- or post expositon with the virus in question, like SARS-CoV, including SARS-CoV-2.

For example, the individual may, or may be suspected of being infected with the coronaviridae. In cases of actual or even suspected infection, the DIPs according to the present invention may be administered as soon as possible, within 72 hours, like within 24 hours of the individual being infected, or being suspected of being infected. Similarly, individuals can be administered the DIPs according to the present invention In another embodiment, treatment may be effected one to two weeks before of potential exposure to the virus in question. As a precautionary measure if they are shortly to be exposed to the virus mentioned herein.

### Materials and methods

### Cells and viruses

Vero-6 (ATCC CRL-1586) cells were maintained in DMEM medium (Gibco, 4.5 g/L Glucose, w/o Pyruvate) supplemented with 10% fetal calf serum (FCS, Biowest, S1810-6500), 100 IU/mL penicillin, 100 µg/mL streptomycin, 1x GlutaMax (Gibco) and 1x sodium pyruvate (Gibco). Calu-3 (ATCC HTB-55) were cultured in MEM (Sigma) supplemented with 10% FCS (Biowest, S1810-6500), 100 IU/mL penicillin, 100 µg/mL streptomycin, 1x GlutaMax (Gibco) and 1x sodium pyruvate (Gibco). Caco-2 (ATCC HTB-37) were grown in MEM (Gibco) supplemented with 20 % FCS (Biowest, S1810-6500), 100 IU/mL penicillin, 100 µg/mL streptomycin, 1x GlutaMax (Gibco) and 1x non-essential amino acid solution (Gibco). All cells were maintained or infected at 37°C in a 5% CO2 atmosphere.

The IAV DIPs DI244 and OP7 were produced in a cell culture-based process using a 500 mL laboratory-scale stirred tank bioreactor, followed by purification and concentration by steric exclusion chromatography (Marichal-Gallardo et al., 2017), as described previously (Hein et al., 2020). Production titers of 3.3 and 3.67 log haemagglutination (HA) units/100µL and 5.6 x 108 and 1.12 x 1011 DI vRNAs/mL were achieved for DI244 and OP7, respectively.

The SARS-CoV-2 isolate hCoV-19/Croatia/ZG-297-20/2020 was used. All experiments with infectious SARS-CoV-2 were performed in the BSL-3 facility at the Helmholtz Centre for Infection Research (Braunschweig, Germany). The SARS-CoV-2 seed virus was produced in Caco-2 cells, and virus particles were enriched in Vivaspin 20 colums (Sartorius Stedim, Biotech) via centrifugation. Collected virus was stored at -80°C. SARS-CoV-2 titers were quantified by plaque assay.

### Plaque assay

Quantification of SARS-CoV-2 was performed by plaque assay. Samples were serially diluted in 10-fold steps, and used to infect a confluent monolayer of Vero-6 cells (on 96-well plates) for 1 h. Then, the inoculum was removed and cells were overlaid with cell culture medium containing 1.5% methyl-cellulose (SIGMA, #C9481-500). At 3 dpi, cells were fixed with 6% formaldehyde and stained with crystal violet. Wells were imaged using a Sartorius IncuCyte S3 (4x objective, whole-well scan) and plaque counts were determined.

### SARS-CoV-2 infection and antiviral treatment

Confluent Calu-3 cells in 96-well plates (∼6 x 104 cells/well) were infected with SARS-CoV-2 (2000 PFU per well). At 1 or 24 hpi, we added active or inactive IAV DIPs (DI244 or OP7) at indicated fractions (% v/v) with respect to the cell culture volume of 100 µL. Whenever indicated, we additionally added 0.8 µM ruxolitinib (Cayman Chemical, Cat. #Cay11609-1) to these wells. Alternatively, remdesivir or IFN-β-1A (instead of IAV DIPs) were added at indicated concentrations at 1 hpi. Supernatants were collected at 3 dpi. Quantification of SARS-CoV-2 titers was performed using the plaque assay.

### Immunofluorescence staining

SARS-CoV-2 infected cells were fixed with 6% paraformaldehyde in PBS for one hour at room temperature, followed by washing with PBS. Cells were permeabilised with 0.1% Triton X-100 in PBS for 10 min at room temperature, washed with PBS, and blocked with 2% BSA in PBS for one hour. Antibody labelling was performed with mouse anti-SARS-CoV-2 S protein (Abcalis, clone AB68-A09, #ABK68-A09-M) and secondary antibody anti-mouse Alexa488 (Cell Signaling Technology, #4408), each step followed by three washing steps with PBS containing 0.05% Tween-20. Finally, cells were overlaid with Vectashield mouting medium (Biozol, #VEC-H-1000).

### Results

### SARS-CoV-2 replication is abrogated by IAV DIP treatment in vitro

In order to test the potential antiviral efficacy of IAV DIPs with regard to the replication of SARS-CoV-2, we conducted *in vitro* co-infection experiments. For this, confluent Calu-3 cells, cultivated in 96-well plates (∼60,000 cells), were infected with 2,000 plaque-forming units (PFU) of SARS-CoV-2. After 1h, we added 10% (v/v, with respect to the culture volume of 100µL) of DI244, or OP7 of highly concentrated DIPs derived from cell culture-based manufacturing (Hein et al., 2020)(Hein et al., submitted), corresponding to 5.6 x 10⁶ and 1.12 x 10⁹ defective interfering (DI) viral RNAs (vRNAs) for DI244 and OP7, respectively. At 3 days post infection (dpi), cells were stained for the SARS-CoV-2 spike protein expression on the cell surface. Indeed, spike protein expression was significantly reduced for cells co-treated with DI244 or OP7 compared to cells infected with SARS-CoV-2 only, indicating suppression of SARS-CoV-2 replication by DIP co-infection.

Next, a range of different concentrations of DIPs was tested for the treatment of SARS-CoV-2-infected cells (Fig. 1A). As a read-out for SARS-CoV-2 replication, we determined the plaque titer from supernatants at 3 dpi using Vero cells (African green monkey kidney epithelial cells). Please note that infection with only defective, replication-incompetent IAV DIPs did not result in the release progeny virions, as demonstrated by negative plaque titers. Surprisingly, SARS-CoV-2 replication was severely diminished upon IAV DIP co-infection. In particular, at the two highest concentrations tested for both DIPs, plaque titers of SARS-CoV-2 were not detectable anymore (limit of detection (LOD) = 33 PFU/mL). Suppression of SARS-CoV-2 replication decreased with increasing dilution of DIPs. Remarkably, though, the treatment of DIPs using a dilution of 1:1000 still resulted in a pronounced inhibition of SARS-CoV-2 replication. For comparison, IFN-β or remdesivir treatment of SARS-CoV-2 infected cells were tested. Both agents were also able to diminish SARS-CoV-2 plaque titers to below the LOD, up until a specific concentration. Yet, inhibiting effects decreased significantly faster with increasing dilutions, most apparently observed for remdesivir.

Fig. 1B illustrates SARS-CoV-2 inhibition caused by inactivated DIPs. These DIPs were previously treated with UV irradiation, until no interfering efficacy (against IAV replication) was observed anymore *in vitro* (Hein et al., 2020), indicative for complete inactivation of the causative interfering agent, i.e. the DI vRNA. In contrast, inhibition of SARS-CoV-2 replication by inactivated DIPs was still detectable (Fig. 1B). More specifically, we still observed a residual suppression of plaque titers. This may be explained by the unspecific stimulation of innate immunity by inactive viral particles, which might have resulted in suppression of SARS-CoV-2 replication. Nevertheless, as inhibition caused by active DIPs was stronger, it indicates to a specific activity of IAV DIPs, leading to interference with SARS-CoV-2 replication. Finally, it is noted that administration of active DIPs even 24 h after SARS-CoV-2 infection still resulted in a pronounced antiviral effect (Fig. 1C).

In conclusion, treatment with both DIPs completely abolished SARS-CoV-2 replication, similar to IFN-β and remdesivir treatment, but antiviral effects of IAV DIPs were significantly more sustained with increasing dilution.

### Inhibition of SARS-CoV-2 replication by IAV DIPs caused by stimulation of innate immunity

Next, to investigate the hypothesis whether inhibition of SARS-CoV-2 replication by DIPs was caused by their ability to stimulate the IFN system, ruxolitinib was used in co-infection experiments. This small molecule drug is an efficient inhibitor of janus kinases (JAK), which are key effectors in the IFN system. JAKs typically recruit signal transducers and activators of transcription (STATs), ultimately leading to the upregulation IFN-stimulated gene (ISGs, effectors that limit viral replication).

Fig. 2 shows the results of SARS-CoV-2 and IAV DIP co-infection upon treatment with ruxolitinib. While DIP co-infection (using both DIPs at a concentration of 20% and 2%) decreased SARS-CoV-2 plaque titers to around the LOD of the assay (33 PFU), additional treatment with ruxolitinib completely abrogated the suppression of SARS-CoV-2 replication caused by IAV DIPs. Specifically, virus titers were insignificantly different as compared to the control SARS-CoV-2 infection without DIPs (and without ruxolitinib). In conclusion, these results suggest a major role of the induction of the innate immune response by IAV DIPS in interfering with SARS-CoV-2 replication.

### Discussion

New antiviral treatment options are needed for COVID-19. Herein it is shown that cell culture-derived, manufactured IAV DIPs are potent inhibitors of SARS-CoV-2 replication. In addition, *in vitro* experiments suggest that suppression of SARS-CoV-2 replication by IAV DIPs can be ascribed to their ability to stimulate innate immunity. The already approved antiviral treatment options for COVID-19 show only very limited efficacy. For instance, treatment with the polymerase inhibitor remdesivir resulted in no overall decrease in mortality in clinical trials (Beigel et al., 2020, Pan et al., 2020). However, for patients receiving supplemental oxygen, an improvement in the survival rate from ∼12 to ∼4% was observed (Beigel et al., 2020). In addition, the time required for recovery of COVID-19 patients decreased by five days (Beigel et al., 2020). Another strategy comprises the use of monoclonal antibodies that target the receptor binding domain of the SARS-CoV-2 spike protein, thereby inhibiting engagement with the host cell entry receptor angiotensin-converting enzyme 2 (ACE2). It was suggested to use antibody cocktails to prevent the emergence of viral escape variants. In clinical trials, treatment of outpatients with one such antibody cocktail (i.e. bamlanivimab) accelerated the decrease in viral load and reduced the fraction of patients requiring hospitalization from 6.3% to 1.6% (Chen et al., 2020b). The clinical use of the corticosteroid dexamethasone results in an overall lower mortality in critically ill COVID-19 patients (e.g. Horby et al., 2020). This has a caveat, though, as a decrease in mortality was observed for patients requiring oxygen (including mechanical ventilation), but increase in mortality for patients not requiring oxygen.

To date, treatment of COVID-19 patients with IFNs has not been approved yet. In general, SARS-CoV-2 infection modulates and inhibits the IFN response (e.g. Chen et al., 2020a). Moreover, it was recently shown that the host cell entry receptor ACE2 is indeed an ISG, and it was speculated that SARS-CoV-2 may exploit the IFN-driven upregulation of ACE2 to enhance infection. However, SARS-CoV-2 replication was also shown to be susceptible to inhibition by exogenously added IFN. For instance, all IFNs (type I, II and III) exhibited potent antiviral activity with SARS-CoV-2 replication *in vitro,* suggesting that the antiviral activities of IFNs may counterbalance any proviral effects derived from ACE2 induction. In agreement with this, in a placebo-controlled phase 2 clinical trial, administration of inhaled, nebulized IFN beta-1a resulted in a higher chance of disease improvement and a more rapid recovery from COVID-19 (Monk et al., 2020).

The IAV DIPs completely abrogated SARS-CoV-2 replication in our *in vitro* studies. However, the UV-irradiated, inactive DIP material also showed a residual inhibitory effect. Yet, the observation of a much stronger antiviral effect upon treatment with active DIPs hints to a specific activity of IAV DIPs in the context of SARS-CoV-2 suppression. DIPs are defective in virus replication, and their infection cannot result in a complete infection cycle. However, the incoming genomic vRNAs, packaged into a viral ribonucleoprotein (vRNP) complex, still show polymerase activity and can still transcribe and produce viral mRNAs. In particular, the short DI vRNAs (and likely, also the resulting short DI mRNAs) were shown to be preferentially bound by the retinoic acid inducible gene I (RIG-I) protein, which subsequently leads to the activation of the IFN-response.

The present results support the notion that IAV DIPs may protect against many other heterologous IFN-sensitive respiratory viruses.

## Claims

1. Influenza virus defective interfering particles (DIPs) for use in the prophylactic or therapeutic treatment of *coronaviridae* infection in an individual.

2. The influenza virus defective interfering particles for use according to claim 1, wherein the influenza virus defecting interfering particles are influenza A virus defective interfering particles.

3. The influenza virus defective interfering particles for use according to claim 1 or 2 for the prophylactic or therapeutic treatment of *orthocoronaviridae*, in particular, *betacoronaviridae.*

4. The influenza virus defective interfering particles for use according to any one of the preceding claims for preventing or treating infection by *sarbecovirus* virus, in particular, *severe acute respiratory syndrome related coronavirus* or of *merbecovirus* including *Middle East respiratory syndrome related coronavirus.*

5. The influenza virus defective interfering particles for use according to any one of the preceding claims in the prophylactic or therapeutic treatment of *severe acute respiratory syndrome related to coronavirus* 2 (SARS-CoV-2) infection.

6. The influenza A virus defective interfering particles for use according to any one of the claims 2 - 5, wherein the defective interfering influenza A virus is a virus,
i) wherein the nucleic acid sequence of segment 1 of the influenza A virus comprises:
a. A sequence collected from SEQ ID No. 1 or SEQ ID No. 2; or
b. a nucleic acid sequence of at least 99 % identity with SEQ ID No. 1 or SEQ ID No. 2; or
ii) wherein the nucleic acid sequence comprises SEQ ID No. 3 containing the following substituents: C3T, G4A, G8A, A100G, G113A, G130A, G240A, A241G, C334T, C353T, C361T, C370A, T371G, T385C, A401T, G434A, C442T, A443G, C453T, A454G, A524G, T643G, G645T, A648G, A667G, G670A, A716G, C793T, G801T, A805G, G874T, A887T, C888T, G894A, G943A, A18G, C535T,compared to the wild type sequence of SEQ ID No. 4; or nucleic sequence having greater than 99 % identity with the sequence of SEQ ID No. 3.

7. The influenza A virus defective interfering particles for use according to any one of the claim 6, wherein the nucleic acid sequence comprises SEQ ID No. 3; or a nucleic acid sequence having greater than 99 % identity with the sequence of SEQ ID No. 3

8. Pharmaceutical composition containing an influenza virus defective particles for use according to any one of claims 1 to 7.

9. The pharmaceutical composition according to claim 8 for use in a therapeutic or prophylactic treatment.

10. The pharmaceutical composition according to claim 8 or 9 being a pharmaceutical composition for mucosal administration, like administration to the respiratory tract by droplet spray or nebulizer.

11. The pharmaceutical composition according to any one of claims 8 to 10 for intranasal administration.

12. The pharmaceutical composition according to any one of claims 8 to 11 for use in stimulating innate immunity against the virus in an individual.

13. A composition or kit comprising an influenza virus defective interfering particles according to any one of claims 1 to 7 or a pharmaceutical composition according to any one of claims 8 to 12, in particular, in form of a pharmaceutical composition, in particular, for use in treating or protecting against a viral infection as defined in any one of claims 1 to 7.

14. Influenza virus defective interfering particles for use according to any one of claims 1 to 7, wherein said virus particle demonstrates after infection at least one of
i). a reduction in the total number of virus particles,
ii). a severe reduction in the faction of infectious virus produced,
iii). a stronger induction of the innate immune response expressed by type I IFN expression is observable.

15. The influenza virus defective interfering particles for use according to any one of claims to 1 to 7, **characterized in, that**, the individuals to be treated is an animal or human, in particular, the animal is selected from cat, ferrets, dog or mink.
